(19) Europäisches Patentamt
European Patent Office
Office européen des brevets

(11)  **EP 2 606 863 B1**

## (12) EUROPÄISCHE PATENTSCHRIFT

(45) Veröffentlichungstag und Bekanntmachung des Hinweises auf die Patenterteilung:
**23.03.2016  Patentblatt 2016/12**

(51) Int Cl.:
*A61F 9/008* *(2006.01)*      *B23K 26/04* *(2006.01)*

(21) Anmeldenummer: **11010104.5**

(22) Anmeldetag: **22.12.2011**

(54) **Vorrichtung und Verfahren zum Bestimmen der Fokusposition eines Laserstrahls**

Device and method to determine the focus position of a laser beam

Dispositif et procédé destinés à la détermination de la position du foyer d'un rayon laser

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**26.06.2013  Patentblatt 2013/26**

(73) Patentinhaber: **Ziemer Ophthalmic Systems AG**
**2562 Port (CH)**

(72) Erfinder: **Rathjen, Christian**
**28197 Bremen (DE)**

(74) Vertreter: **Rentsch Partner AG**
**Rechtsanwälte und Patentanwälte**
**Fraumünsterstrasse 9**
**Postfach 2441**
**8022 Zürich (CH)**

(56) Entgegenhaltungen:
**EP-A1- 1 034 755      DE-A1- 10 349 296**
**US-A1- 2004 070 761**

EP 2 606 863 B1

**Beschreibung**

Technisches Gebiet

Die vorliegende Erfindung betrifft eine Vorrichtung und ein Verfahren zum Bestimmen der Fokusposition eines Laserstrahls in einem ophthalmologischen Laserprojektionssystem. Die vorliegende Erfindung betrifft insbesondere eine Vorrichtung und ein Verfahren zum Bestimmen der Fokusposition in Projektionsrichtung des Laserstrahls.

Stand der Technik

In der Ophthalmologie werden gepulste Laserstrahlen, beispielsweise Laserpulse mit einer Pulsdauer im Femtosekundenbereich ($1fs=10^{-15}s$), zur Behandlung von Augengewebe verwendet. Dabei werden Schnitte durch fokussierte Projektion der Laserpulse ins Augengewebe durchgeführt. Aufgrund von Fertigungstoleranzen und/oder thermischen Ausdehnungen bei der Herstellung respektive dem Betrieb von ophthalmologischen Laserprojektionssystemen ist die genaue Fokusposition der Laserpulse in Projektionsrichtung bezüglich dem ophthalmologischen Laserprojektionssystem, insbesondere in Bezug zum Projektionsobjektiv und/oder einem Kontaktkörper, durch den die Laserpulse ins Gewebe projiziert werden, in der Regel nicht genügend genau bekannt. Selbst wenn die Fokusposition durch Kalibrierung in der Fertigung des Laserprojektionssystems bestimmt werden kann, ist es möglich, dass sich diese Position im Betrieb ändert, beispielsweise bei thermischen Materialausdehnungen oder Auswechseln des verwendeten Kontaktkörpers.

Dokument DE 103 49 296 offenbart einen Adapter zum Koppeln einer Laserbefarbeitungsvorrichtung mit einem Objekt. Dabei wird eine auf den Adapter aufgebrachte Referenzstruktur mittels Laserstrahlung detektiert.

In EP 2 069 099 wird ein Verfahren zum konfokalen Detektieren der Eintrittsfläche und Austrittsfläche eines Kontaktkörpers beschrieben. Gemäss EP 2 069 099 wird ein Lasermessstrahl mittels einer variablen Fokusverstelleinrichtung möglichst auf die Flächen fokussiert und rückgestreute oder reflektierte Strahlung werden konfokal detektiert. Aus der konfokal detektierten Strahlung und der zugeordneten Einstellung der variablen Fokusverstelleinrichtung wird die Lage der Flächen bestimmt. Das Verfahren gemäss EP 2 069 099 erfordert eine Fokussierbewegung für die Bestimmung der Flächen, was die Messgenauigkeit beeinträchtigen kann. Überdies liefert die konfokale Detektion sehr schwache Signale, so dass für genaue Messungen ein hoher messtechnischer Aufwand erforderlich ist.

Darstellung der Erfindung

Es ist eine Aufgabe der vorliegenden Erfindung eine Vorrichtung und ein Verfahren zum Bestimmen der Fokusposition eines Laserstrahls in einem ophthalmologischen Laserprojektionssystem vorzuschlagen, welche zumindest einige Nachteile der bekannten Systeme nicht aufweisen. Es ist insbesondere eine Aufgabe der vorliegenden Erfindung eine Vorrichtung und ein Verfahren zum Bestimmen der Fokusposition in Projektionsrichtung des Laserstrahls vorzuschlagen, welche während des Messvorgangs ohne Fokussierbewegung auskommen.

Gemäss der vorliegenden Erfindung werden diese Ziele durch die Merkmale der unabhängigen Ansprüche erreicht. Weitere vorteilhafte Ausführungsformen gehen ausserdem aus den abhängigen Ansprüchen und der Beschreibung hervor.

Die oben genannten Ziele werden durch die vorliegende Erfindung insbesondere dadurch erreicht, dass zum Bestimmen der Fokusposition eines Laserstrahls in einem ophthalmologischen Laserprojektionssystem mindestens eine auf einer Referenzfläche, insbesondere auf einer Referenzebene angebrachte Messmarkierung mit dem Laserstrahl entlang einer Abtastbahn überfahren wird, dass ein durch das Überfahren der Messmarkierung erzeugtes Messsignal erfasst wird, dass Zeitwerte von mindestens einer im Messsignal beim Überfahren von Kanten der Messmarkierung entstehenden Signalflanke bestimmt werden, und dass die Fokusposition basierend auf den Zeitwerten bestimmt wird. Durch das Abtasten von definierten Messmarkierungen und der Ermittlung von Zeitwerten von Signalflanken, die beim Überfahren von Kanten der Messmarkierung entstehen, kann die Fokusposition des Laserstrahls bestimmt werden, ohne dass dazu Fokussierbewegungen während der Messung notwendig sind.

In einer Ausführungsvariante ist die Messmarkierung fotoaktiv ausgestaltet und beim Überfahren der Messmarkierung mit dem Laserstrahl erzeugt die Messmarkierung das Messsignal. In einer alternativen Ausführungsvariante ist die Messmarkierung reflektierend ausgestaltet und das Messsignal wird aufgrund eines beim Überfahren der Messmarkierung reflektierten Laserstrahls erzeugt. In einer weiteren alternativen Ausführungsvariante ist die Messmarkierung absorbierend ausgestaltet und das Messsignal wird aufgrund eines beim Überfahren der Messmarkierung absorbierten respektive nicht reflektierten oder nicht transmittierten Laserstrahls erzeugt.

In einer Ausführungsvariante wird basierend auf den Zeitwerten die Strahlbreite bestimmt und basierend auf der Strahlbreite wird die Fokusposition in Projektionsrichtung des Laserstrahls ermittelt.

In einer Ausführungsvariante wird in der Signalflanke eine Zeitdauer zwischen einem oberen und einem unteren Signalschwellwert bestimmt, die Strahlbreite wird basierend auf der Zeitdauer bestimmt und basierend auf der Strahlbreite wird die Fokusposition in Projektionsrichtung des Laserstrahls ermittelt.

In einer Ausführungsvariante ist die Messmarkierung mit einem Spalt respektive einem Balken bekannter Breite ausgestaltet. Der Spalt respektive der Balken wird auf einer quer zum Spalt respektive zum Balken verlaufenden Abtastbahn überfahren. Aus dem Messsignal wird eine Zeitdauer für das Überfahren der Breite des Spalts respektive des Balkens bestimmt und die Fokusposition wird basierend auf dieser Zeitdauer und der Breite des Spalts respektive des Balkens ermittelt. Bei einer Messmarkierung mit einem Spalt respektive einem Balken werden nacheinander zwei Kanten mit bekannter Distanz überfahren, so dass die aktuelle Geschwindigkeit bestimmt werden kann, mit der die Messmarkierung abgetastet wird. Somit wird die Bestimmung der Fokusposition auch ermöglicht, wenn die Abtastgeschwindigkeit variabel und unbekannt ist. Je näher die Kanten respektive die dadurch entstehenden Signalflanken beieinander liegen, umso genauer ist die Messung auch bei variabler Abtastgeschwindigkeit.

In einer weiteren Ausführungsvariante sind mehrere Messmarkierungen auf der Referenzfläche auf einem Kreis angeordnet. Die Messmarkierungen sind jeweils mit mindestens einer im Wesentlichen senkrecht zum Kreis verlaufenden ersten Kante und einer schräg zu dieser ersten Kante verlaufenden zweiten Kante ausgestaltet. Die kreisförmig angeordneten Messmarkierungen werden auf einer kreisförmigen oder spiralförmigen Abtastbahn überfahren. Von den im Messsignal beim Überfahren der ersten Kante und der zweiten Kante entstehenden Signalflanken werden Zeitwerte bestimmt und aus den Zeitwerten wird ein Zentrierkennwert bezüglich der Zentrierung der kreisförmigen oder spiralförmigen Abtastbahn zu den kreisförmig angeordneten Messmarkierungen ermittelt.

Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:

Figur 1: zeigt ein Blockdiagram, welches schematisch ein ophthalmologisches Laserprojektionssystem mit einer Vorrichtung zum Bestimmen der Fokusposition des Laserstrahls illustriert.

Figur 2: zeigt ein Blockdiagram, welches schematisch die Vorrichtung zur Bestimmung der Fokusposition des Laserstrahls illustriert.

Figur 3: zeigt in einer Aufsicht ein Beispiel einer Messmarkierung (rechts) und eine Anordnung von mehreren Messmarkierungen auf einer Referenzfläche eines Körpers (links).

Figur 4: zeigt ein Beispiel eines Messsignals mit Signalflanken, die beim Überfahren von Kanten der Messmarkierung mit dem Laserstrahl entstehen.

Figur 5: zeigt ein Beispiel einer Signalflanke des Offset-korrigierten Messsignals und Messpunkte an einem oberen und unteren Signalschwellwert zur Bestimmung von Zeitwerten und einer darauf basierenden Zeitdauer der Signalflanke.

Figur 6: zeigt am Beispiel eines Offset-korrigierten Messsignals mehrere Messpunkte zur Bestimmung von Zeitwerten im Messsignal.

Figur 7: zeigt im Querschnitt einen Laserstrahl mit gaussschem Strahlprofil.

Wege zur Ausführung der Erfindung

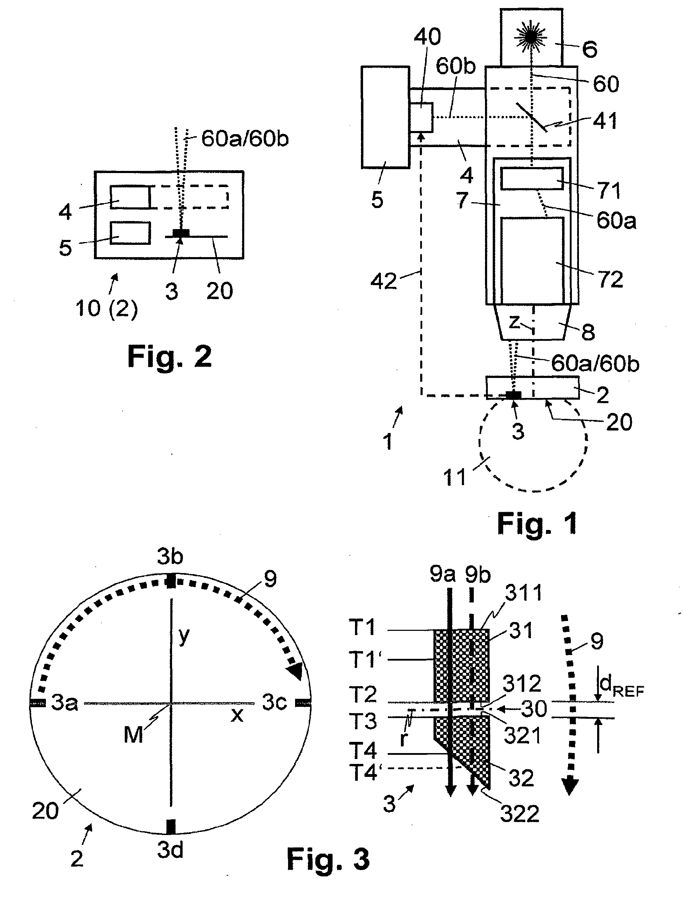

In der Figur 1 bezieht sich das Bezugszeichen 1 auf ein ophthalmologisches Lasersystem mit einer Laserquelle 6 zur Erzeugung eines Laserstrahls 60. Die Laserquelle 6 ist eingerichtet einen gepulsten Laserstrahl 60 zu erzeugen, insbesondere Femtosekundenlaserpulse (1fs= $10^{-15}$s). Der Laserstrahl 60 wird über ein optisches Übertragungssystem 7 von der Laserquelle 6 zur Projektionsoptik 8 übertragen, welche eingerichtet ist, den Laserstrahl 60 respektive dessen Laserpulse fokussiert ins Augengewebe 11 zu projizieren, beispielsweise in die Cornea oder die Augenlinse, etc.. Neben optischen Elementen 72, wie Linsen und Spiegeln, umfasst das optische Übertragungssystem 7 zudem einen strahlablenkenden Scanner 71. Zum Ablenken des Laserstrahls 60 umfasst der Scanner 71 vorzugsweise einen beweglichen Spiegel und ist beispielsweise als Galvanoscanner, Piezoscanner, Polygonscanner oder Resonanzscanner ausgeführt. Der abgelenkte Laserstrahl 60a wird über die optischen Elemente 72 des Übertragungssystems 7 und die Projektionsoptik 8 auf respektive in das Augengewebe 11 projiziert und durch den Scanner 71 entlang einer definierten Abtastbahn bewegt. In einer alternativen Variante umfasst der Scanner 71 einen oder mehrere Antriebe zur mechanischen Bewegung der Projektionsoptik 8 oder Teilen der Projektionsoptik 8.

Zur Bestimmung der Fokusposition des Laserstrahls 60 ist eine Vorrichtung 10 (siehe auch Figur 2) vorgesehen, welche eine oder mehrere Messmarkierungen 3, 3a, 3b, 3c, 3d aufweist (siehe auch Figur 3), die auf einer Referenzfläche 20, insbesondere auf einer Referenzebene, eines Körpers 2 angeordnet sind.

Wie in der Figur 2 schematisch dargestellt ist umfasst die Vorrichtung 10 zur Bestimmung der Fokusposition des Laserstrahls 60 überdies ein Detektionssystem 4 und ein damit verbundenes Verarbeitungssystem 5.

Wie in der Figur 1 schematisch dargestellt ist, ist der Körper 2 in einer Variante als transparenter oder semitransparenter Kontaktkörper ausgestaltet, der während der Behandlung des Augengewebes 11 am Auge angebracht ist, z.B. mittels eines Saugrings. In einer alternativen Variante ist der Körper 2 dem Auge vorgeschaltet ohne dieses zu berühren, beispielsweise fest im Strahlengang oder temporär in den Strahlengang einsetzbar, einschiebbar oder einschwenkbar. Die Messmarkierungen 3, 3a, 3b, 3c, 3d sind je nach Ausführungsvariante auf einer Referenzfläche 20 angeordnet, die auf einer dem Auge zugewandten Oberfläche des Körpers 2, auf einer vom Auge abgewandten Oberfläche des Körpers 2, oder innerhalb des Körpers 2 vorzugsweise senkrecht zur Projektionsachse z der Projektionsoptik 8 angeordnet ist. In einer weiteren Ausführungsvariante ist der Körper 2 als Kunstauge ausgeführt, d.h. er ist in der Form eines menschlichen Auges ausgestaltet.

Die Messmarkierungen 3, 3a, 3b, 3c, 3d sind in unterschiedlichen Ausführungsvarianten reflektierend (respektive absorbierend) oder fotoaktiv ausgestaltet. In der reflektierenden Variante wird ein auf die Messmarkierung 3, 3a, 3b, 3c, 3d projizierter Laserstrahl 60a von der Messmarkierung 3, 3a, 3b, 3c, 3d reflektiert. Der reflektierte Laserstrahl 60b wird vom Detektionssystem 4 erfasst, beispielsweise über einen Umlenkspiegel 41, einem Signalwandler 40 zugeführt und als Messsignal in einem Datenspeicher aufgezeichnet. Umgekehrt wird bei einer absorbierenden Massmarkierung 3, 3a, 3b, 3c, 3d vom Detektionssystem 4, ein absorbierter Laserstrahl detektiert, beispielsweise aufgrund einer fehlenden Reflexion oder Transmission des Laserstrahls 60, 60a. Bei der Detektion einer aufgrund einer absorbierenden Markierung 3, 3a, 3b, 3c, 3d fehlenden Transmission des Laserstrahls 60, 60a durch den Körper 2 erfasst das Detektionssystem 4 den transmittierten Laserstrahl 60, 60a auf der vom Projektionsoptik 8 abgewandten Seite des Körpers 2 respektive der Referenzfläche 20. Der Signalwandler 40 umfasst beispielsweise einen Fotosensor und einen A/D-Wandler. Die reflektierenden Messmarkierungen 3, 3a, 3b, 3c, 3d umfassen spiegelnd und diffus reflektierende Materialien. Die reflektierenden Messmarkierungen 3, 3a, 3b, 3c, 3d sind beispielsweise als Chrommasken, Blenden oder teiltransparente, dichroidische Beschichtungen ausgeführt. In einer weiteren Variante sind die Messmarkierungen 3, 3a, 3b, 3c, 3d wellenselektiv ausgestaltet, so dass sie Laserstrahlen 60, 60a einer ersten Wellenlänge zur Bearbeitung des Augengewebes 11 transparent durchlassen und Laserstrahlen 60, 60a einer zweiten Wellenlänge zur Bestimmung der Fokusposition reflektieren respektive absorbieren. In der fotoaktiven Variante erzeugt die Messmarkierung 3, 3a, 3b, 3c, 3d aufgrund eines aufprojizierten Laserstrahls 60a ein Messsignal, das vom Detektionssystem 4 erfasst und über eine Signalleitung 42 dem Signalwandler 40 zur digitalisierten Aufzeichnung im Datenspeicher zugeführt wird, wie in der Figur 1 durch den gestrichelt markierten Pfeil angedeutet ist. Die fotoaktiven Messmarkierungen 3, 3a, 3b, 3c, 3d sind beispielsweise als Fotosensoren zur Erzeugung eines Fotostroms oder als Fotowiderstände mit lichtabhängigem Widerstand ausgeführt.

Das Verarbeitungssystem 5 ist eingerichtet, basierend auf dem erfassten Messsignal die Fokusposition des Laserstrahls 60, 60a zu ermitteln, d.h. die aktuelle Lage des Fokus des Laserstrahls 60, 60a in Projektionsrichtung z' respektive als Relativwert auf der Projektionsachse z in Bezug zur Projektionsoptik 8 oder anderen Referenzpunkten des ophthalmologischen Lasersystems 1. Die Funktionalität und Funktionsweise des Verarbeitungssystems 5 wird später detaillierter beschrieben. Das Verarbeitungssystem 5 ist als Logikmodul ausgeführt und umfasst je nach Ausführungsvariante einen Prozessor und programmierte Softwaremodule mit gespeichertem Programmcode zur Steuerung des Prozessors, oder eine programmierte Hardware Logik. Der Fachmann wird verstehen, dass das Verarbeitungssystem 5 in einer weiteren Ausführungsvariante auch mit (elektronischen) Hardwarekomponenten ausgeführt werden kann.

In einer Ausführungsvariante ist die Vorrichtung 10 zur Bestimmung der Fokusposition des Laserstrahls 60 als Teil des ophthalmologischen Lasersystems 1 ausgeführt, wie in der Figur 1 schematisch dargestellt ist. In einer alternativen Ausführungsvariante ist die Vorrichtung 10 zur Bestimmung der Fokusposition des Laserstrahls 60, 60a im respektive als Körper 2 ausgestaltet, d.h. zusätzlich zu der Referenzfläche 20 und den darauf angeordneten Messmarkierungen 3, 3a, 3b, 3c, 3d umfasst der Körper 2 überdies das Detektionssystem 4 und das Verarbeitungssystem 5, wie in der Figur 2 mit dem in Klammern angefügten Bezugszeichen 2 angedeutet ist.

Die Figur 3 zeigt auf der rechten Seite ein Beispiel einer Messmarkierung 3, welche vom Laserstrahl 60, 60a auf einer oder mehreren Abtastbahnen 9a, 9b in der dargestellten Pfeilrichtung überfahren (abgetastet) wird. Wie in der Figur 3 ersichtlich ist, weist die Messmarkierung 3 einen Spalt 30 mit einer definierten Breite $d_{REF}$ auf, welcher vom Laserstrahl auf einer Abtastbahn 9, 9a, 9b überfahren wird. Die Breite $d_{REF}$ beträgt beispielsweise 40 $\mu$m bis 100$\mu$m, z.B. 60 $\mu$m. Der Spalt 30 unterscheidet sich vom übrigen Teil der Messmarkierung 3 dadurch, dass er nicht reflektierend (oder absorbierend) respektive nicht fotoaktiv ist. Der Fachmann wird verstehen, dass in einer alternativen Ausführungsvariante anstelle eines Spalts ein Balken mit definierter Breite $d_{REF}$ vorgesehen werden kann, der aus dem reflektierenden (oder absorbierenden) respektive fotoaktiven Material ausgeführt ist. Beim Überfahren des Spalts 30 respektive eines entsprechenden Balkens überschreitet der Laserstrahl 60 zwei parallele Kanten 312, 321 der Messmarkierung 3, welche den Spalt 30 respektive den Balken quer zur Abtastbahn 9, 9a, 9b definieren respektive begrenzen. An diesen Kanten 312, 321 weist ein durch das Überfahren der Messmarkierung 3 erzeugtes Messsignal jeweils eine Signalflanke mit einem ansteigenden respektive abfallenden Signalwert auf, da die Messmarkierung 3 an dieser Stelle eine Grenze zwischen reflektierendem und nicht reflektierendem (oder absorbierendem und nicht absorbierendem) respektive fotoaktivem und nicht fotoaktivem Material aufweist.

Bei der in Figur 3 rechts abgebildeten Messmarkierung 3 wird der Spalt 30 durch einen dem Spalt 30 vorgelagerten Eingangsbereich 31 und einen dem Spalt 30 nachgelagerten Ausgangsbereich 32 begrenzt respektive definiert. Der Eingangsbereich 31 weist eine rechteckige Form mit einer Eingangskante 311 und einer den Spalt 30 begrenzenden Ausgangskante 312 auf, welche im Wesentlichen senkrecht zur Abtastbahn 9, 9a, 9b verlaufen. Der Ausgangsbereich 32 weist eine rechtwinklige Trapezoidform mit einer den Spalt 30 begrenzenden, im Wesentlichen senkrecht zur Abtastbahn 9, 9a, 9b verlaufen Eingangskante 321 und einer schräg, beispielsweise 45°, zur Abtastbahn 9, 9a, 9b verlaufenden Ausgangskante 322 auf. Die beiden Seitenränder des Eingangsbereichs 31 und des Ausgangsbereichs 32 verlaufen senkrecht zur Eingangskante 311 und Ausgangskante 312 des Eingangsbereichs 31 und zur Eingangskante 321 des Ausgangsbereichs 32. Die Bezugszeichen T1, T1', T2, T3, T4, T4' bezeichnen Zeitpunkte an denen der Laserstrahl 60, 60a eine betreffende Kante der Messmarkierung 3, 3a, 3b, 3c, 3d überfährt respektive abtastet.

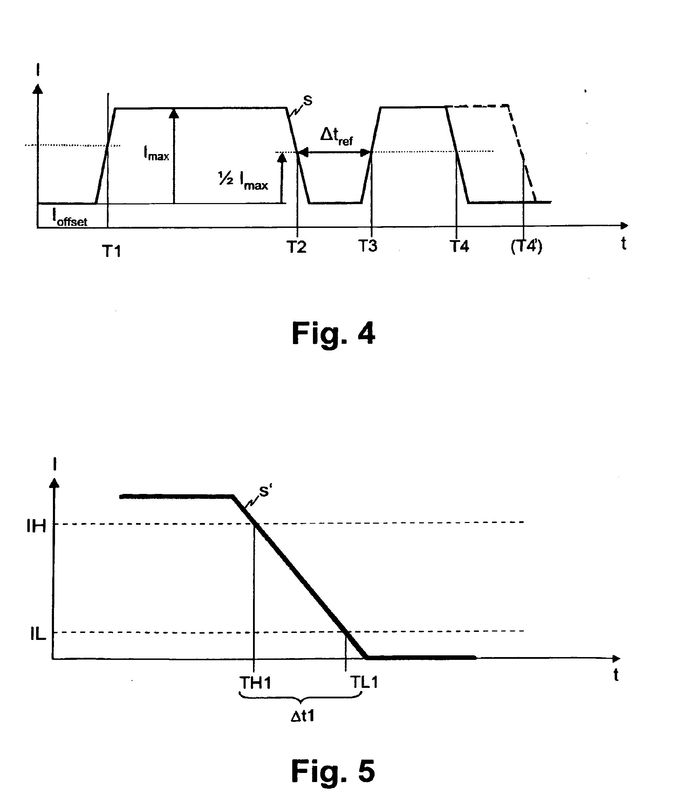

In den nachfolgenden Abschnitten werden die Funktionen des Verarbeitungssystems 5 und das Verfahren zum Bestimmen der Fokusposition des Laserstrahls 60 mit Bezug zu den Figuren 4, 5, 6 und 7 beschrieben.

Beim Einleiten des Messvorgangs wird die Laserquelle 6 in einen Messmodus versetzt, wobei die Leistung der Laserquelle 6 auf eine Messleistung gesetzt wird, welche die Messmarkierungen 3, 3a, 3b, 3c, 3d nicht beschädigt oder zerstört.

Der Scanner 71 wird so angesteuert, dass er die Referenzfläche 20 entlang einer Abtastbahn 9, 9a, 9b abtastet. Wie auf der linken Seite der Figur 3 dargestellt ist, wird die Referenzfläche 20 beispielsweise mit dem Laserstrahl 60, 60a auf einer kreisförmigen Abtastbahn 9 abgefahren. Dabei wird vom Detektionssystem 4 synchron oder asynchron zum Abfahren der Abtastbahn 9, 9a, 9b das durch die Messmarkierungen 3, 3a, 3b, 3c, 3d amplitudenmodulierte Messsignal s erfasst, das auf dem auf der Referenzfläche 20 durch die reflektierenden (oder absorbierenden) Messmarkierungen 3, 3a, 3b, 3c, 3d reflektierten (oder absorbierten) Laserstrahl 60b respektive dem von den fotoaktiven Messmarkierungen 3, 3a, 3b, 3c, 3d erzeugten Signal beruht.

In einer vorbereitenden Korrekturphase ermittelt das Verarbeitungssystem 5 aus dem Signalverlauf des aufgezeichneten Messsignals s, beispielsweise vor Erreichen der Eingangskante 311 zum Zeitpunkt T1 (oder bei einem Eingangsbereich 31 mit unterschiedlicher Länge zum Zeitpunkt T1'), den aktuellen Dunkelwert, d.h. den Signalpegel der auf der Referenzfläche 20 in den Bereichen ausserhalb der Messmarkierungen 3, 3a, 3b, 3c, 3d gemessen wird. Dieser aktuelle Dunkelwert wird wie in der Figur 4 dargestellt als Offsetwert $I_{offset}$ verwendet, um das Messsignal s durch Subtraktion des Offsetwerts $I_{offset}$ vom Signalpegel I zu korrigieren. Anschliessend ermittelt das Verarbeitungssystem 5 in einem definierten Bereich der Messmarkierung 3, beispielsweise im Eingangsbereich 31 zwischen der Eingangskante 311 und der Ausgangskante 312, im Zeitraum zwischen den Zeitpunkten T1 (T1') und T2, den Mittelwert des Messsignals s und daraus den Höchstpegel $I_{max}$ des Messsignals s und normiert das Messsignal s auf den Höchstpegel $I_{max}$. Dabei setzt das Verarbeitungssystem 5 die Schwellwerte des Signalpegels zur Bestimmung der Zeitpunkte T1, T1', T2, T3, T4, T4' des Überfahrens einer Signalflanke bei einer Kante der Messmarkierung 3, 3a, 3b, 3c, 3d auf die Hälfte des Höchstpegels $I_{max}$.

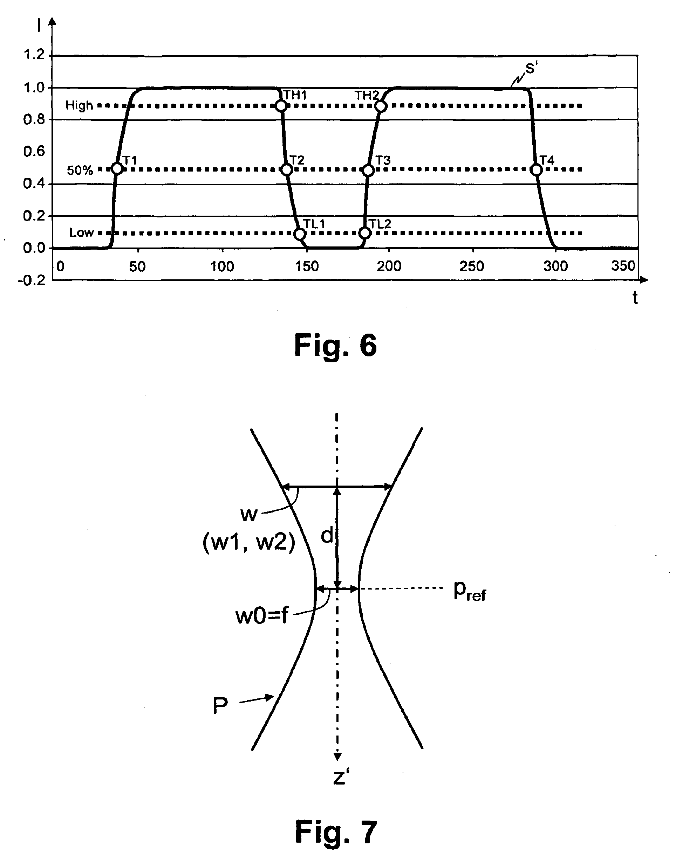

Das Verarbeitungssystem 5 ermittelt die Strahlbreite w (siehe Figur 7) und daraus die Fokusposition basierend auf der Auswertung eines Signalabfalls respektive Signalanstiegs zwischen zwei Schwellwerten IH, IL für einen Hochpegel und einen Tiefpegel des Signalpegels I bei einer abfallenden respektive ansteigenden Signalflanke, die beim Überfahren einer Ausgangskante 312, 322 respektive Eingangskante 311, 321 der Messmarkierungen 3, 3a, 3b, 3c, 3d im Messsignal s, s' entsteht. Die Schwellwerte IH, IL für den Hochpegel und den Tiefpegel werden als Teil des Höchst-pegels $I_{max}$ definiert, z.B. IH= $I_{max}(1-1/e^2)$ und IL= $I_{max}(1/e^2)$, wie in der Figur 5 am Beispiel einer abfallenden Signalflanke illustriert wird. Die Zeitdauer $\Delta t_1$ für die abfallende Signalflanke beim Überfahren der Ausgangskante 312, 322 ergibt sich aus der Differenz der Zeitpunkte TH1, TL1 für die Zeitdauer zwischen dem Erreichen des Hochpegels IH und des Tiefpegels IL des Signalpegels I, wenn die Ausgangskante 312 überfahren wird, $\Delta t_1$=TL1-TH1, wie in der Figur 5 illustriert wird. Die Zeitdauer $\Delta t_2$ für die ansteigende Signalflanke beim Überfahren der Eingangskante 321 ergibt sich entsprechend aus der Differenz der Zeitpunkte TH2, TL2 (siehe Figur 6) für die Zeitdauer zwischen dem Erreichen des Hochpegels IH und des Tiefpegels IL des Signalpegels I, wenn die Eingangskante 321 überfahren wird, $\Delta t_2$=TH2-TL2.

Bei konstanter Abtastgeschwindigkeit reicht die Ermittlung der Zeitdauer $\Delta t_1$, $\Delta t_2$ einer einzigen Signalflanke aus, um die Referenzposition $p_{ref}$ des Fokus F, d.h. die Stelle des projizierten Laserstrahls 60a mit der kleinsten Strahlbreite w0=f, zu bestimmen (siehe Figur 7). Diese Referenzposition $p_{ref}$ entspricht der Position in Projektionsrichtung z' des Laserstrahls 60, 60a, bei der die gemessene Signalflanke die kürzeste Zeitdauer $\Delta t_1$, $\Delta t_2$ aufweist. Zum Auffinden dieser Referenzposition $p_{ref}$ ist eine Fokussierbewegung erforderlich. Anstelle der Ermittlung der Zeitdauer $\Delta t_1$, $\Delta t_2$ der Signalflanke, wird in alternativen Ausführungsvarianten die kürzeste, d.h. steilste, Signalflanke durch Differenzierung der Signalflanke oder durch andere Signalverarbeitungsverfahren (z.B. Sprungantwortfunktionen, Signalbreitenbestimmung über Standardabweichung und Fit einer Fokussierfunktion, etc.) bestimmt und die entsprechende Position in Projektionsrichtung z' als Referenzposition $p_{ref}$ erfasst. In einer Variante werden in einer Tabelle über einen ausgedehnten Bereich mehrere Positionen entlang der Projektionsrichtung z' respektive Projektionsachse z und zugeordnete Strahlbreiten w an diesen Positionen gespeichert, die das Strahlprofil P (siehe Figur 7) des Laserstrahls 60, 60 bestimmen, welches in einer Strahlprofiltabelle oder als gaussche Kurvenfunktion definiert ist.

Die Zeit $\Delta t_{ref}$ für das Überfahren des Spalts 30 respektive des Balkens der Messmarkierung 3, 3a, 3b, 3c, 3d ergibt sich aus der Differenz der Zeitpunkte T2, T3 wenn die Ausgangskante 312 und die Eingangskante 321 überfahren werden, $\Delta t_{ref}$=T3-T2, wie in der Figur 4 illustriert wird.

Das Verarbeitungssystem 5 ermittelt die Zeitdauer $\Delta t_1$ für die abfallende Signalflanke, die Zeitdauer $\Delta t_2$ für die ansteigende Signalflanke und die Zeit $\Delta t_{ref}$ für das Überfahren des Spalts 30 respektive des Balkens mit der definierten Breite $d_{REF}$. Aus der definierten Breite $d_{REF}$ und den bestimmten Zeitdauern $\Delta t_1$, $\Delta t_2$ und $\Delta t_{ref}$ ermittelt das Verarbeitungssystem 5 die Strahlbreite w1 beim Überfahren der Ausgangskante 312 und die Strahlbreite w2 beim Überfahren der Eingangskante 321 gemäss den nachfolgenden Gleichungen (1) und (2):

$$w1 = (\Delta t_1 / \Delta t_{ref}) \, d_{REF} \qquad\qquad (1)$$

$$w2 = (\Delta t_2 / \Delta t_{ref}) \, d_{REF} \qquad\qquad (2)$$

Dabei entspricht $d_{REF}/\Delta t_{ref}$ der aktuellen, lokalen Abtastgeschwindigkeit beim Überfahren des Spalts 30. Die Strahlbreiten w1, w2 liegen beispielsweise im Bereich von $1\,\mu m$ bis $10\,\mu m$. Bei konstanter Abtastgeschwindigkeit weisen die Strahlbreiten w1 und w2 den gleichen Wert auf. Bei sich ändernder Abtastgeschwindigkeit, insbesondere bei sich linear ändernder Abtastgeschwindigkeit, wird vorzugsweise ein Mittelwert der Strahlbreiten w1 und w2 gebildet, der für die Bestimmung der Fokusposition des Laserstrahls 60, 60a verwendet wird.

Aus den bestimmten Strahlbreiten w1, w2, insbesondere aus deren Mittelwert, ermittelt das Verarbeitungssystem 5 die Fokusposition des Laserstrahls 60, 60a auf der Basis des bekannten Strahlprofils P des Laserstrahls 60, 60a, welches beispielsweise in der Strahlprofiltabelle oder einer entsprechenden gaussschen Kurvenfunktion definiert ist. Wie aus der Figur 7 ersichtlich ist, ermittelt sich die Fokusposition des Laserstrahls 60, 60a aus der durch das Strahlprofil P gegebenen Distanz d zwischen der Stelle im Strahlprofil P mit der bekannten Strahlbreite w, w1, w2 und der Stelle $p_{ref}$ im Strahlprofil P, an der die Strahlbreite w0 dem Fokusdurchmesser f entspricht. Wenn a priori nicht sicher gestellt werden kann, in welcher Richtung ausgehend von der ermittelten Strahlbreite w, w1, w2 die Fokusposition $p_{ref}$ mit der kleinsten Strahlbreite w0=f liegt, wird eine zweite Messung mit veränderter Fokussierposition (z-Lage) durchgeführt. Ein iteratives, mehrfach justierendes, insbesondere kontinuierliches Fokussieren während der Messung - wie bei konfokalen Verfahren - ist jedoch nicht erforderlich, in diesem Fall sind einzig zwei Messungen erforderlich.

Wie in der Figur 3 links dargestellt ist, sind in einer Ausführungsvariante mehrere Messmarkierungen 3a, 3b, 3c, 3d auf der Referenzfläche 20 angeordnet, die jeweils wie die in der Figur 3 rechts dargestellte Messmarkierung 3 ausgeführt sind. Die Messmarkierungen 3a, 3b, 3c, 3d sind auf einem Kreis angeordnet, vorzugsweise gleichmässig auf dem Kreis verteilt, z.B. jeweils in den Schnittpunkten des Kreises mit der durch das Zentrum des Kreises verlaufenden Ordinate y und Abszisse x. Dabei sind die Messmarkierungen 3a, 3b, 3c, 3d auf dem Kreis jeweils so angeordnet, dass die Mittelachse r ihres Spalts 30 durch das Zentrum M des Kreises verläuft. Die den Spalt 30 begrenzenden Kanten 312, 321 verlaufen somit im Wesentlichen senkrecht zum Kreis. Die Messmarkierungen 3a, 3b, 3c, 3d sind zudem auf dem Kreis gleich - im Uhrzeigersinn oder Gegenuhrzeigersinn - ausgerichtet und orientiert, so dass sie vom Laserstrahl 60, 60a auf einer (konzentrisch) kreisförmigen (oder spiralförmigen) Abtastbahn 9, 9a, 9b jeweils zunächst über ihren Eingangsbereich 31, danach über den Spalt 30 und dann über ihren Ausgangsbereich 32 abgetastet werden.

Abhängig vom Radius der kreisförmigen (oder spiralförmigen) Abtastbahn 9, 9a, 9b ergeben sich beim Überfahren der schrägen Ausgangskante 322 unterschiedliche Zeitwerte T4, T4' in Bezug zu den Zeitwerten T2, T3 beim Überfahren der vorgelagerten Kanten 312, 321, die den Spalt 30 begrenzen. Je grösser der Bahnradius ist, umso grösser wird der Zeitwert T4, T4', wenn der breitere Seitenrand des trapezoidförmigen Ausgangsbereichs 32 der Messmarkierungen 3, 3a, 3b,3c, 3d vom Zentrum des Kreises abgewandt ist, wie in der Figur 4 gestrichelt dargestellt ist (und umgekehrt).

Gemäss untenstehender Gleichung bestimmt das Verarbeitungssystem 5 aus den Zeitwerten T2, T3, T4, T4' einen Zentrierkennwert ZK, der die relative radiale Position respektive Zentrierung der kreisförmigen oder spiralförmigen Abtastbahn 9 zu den kreisförmig angeordneten Messmarkierungen 3, 3a, 3b, 3c, 3d angibt.

$$ZK=(T3-T2)/(T4-T3) \quad \text{respektive} \quad ZK=(T3-T2)/(T4'-T3) \qquad (3)$$

Bei einem vollständigen Umlauf um die Abtastbahn 9, 9a, 9b wird vom Verarbeitungssystem 5 für jede der mindestens drei Messmarkierungen 3, 3a, 3b,3c, 3d ein Zentrierkennwert ZK ermittelt und gespeichert. Die Zentrierung der Abtastbahn 9, 9a, 9b zur kreisförmigen Anordnung der Messmarkierungen 3, 3a, 3b, 3c, 3d ist dann optimal, wenn die Zentrierkennwerte ZK für sämtliche Messmarkierungen 3, 3a, 3b, 3c, 3d gleich gross sind. Die Zentrierung ist somit unabhängig von der lokalen Geschwindigkeit, mit der der Laserstrahl 60, 60a respektive der Brennpunkt F auf der Abtastbahn 9, 9a, 9b bewegt wird, und vom Absolutwert des Bahnradius, solange dieser im Bereich der Messmarkierungen 3, 3a, 3b,3c, 3d liegt. In einer weiteren Ausführungsvariante ermittelt das Verarbeitungssystem 5 aus den Messwerten mehrerer Messmarkierungen 3, 3a, 3b, 3c, 3d überdies die Verkippung der Referenzfläche 20 gegenüber der Projektionsachse z respektive Projektionsrichtung z'.

Zur Erkennung eines Quadranten ermittelt das Verarbeitungssystem 5 einen Quadrantenmarker QM gemäss untenstehender Gleichung:

$$QM=(T2-T1)/(T3-T2) \qquad (4)$$

Dabei weisen die Markierungen 3, 3a, 3b, 3c, 3d zur Identifizierung der Quadranten respektive Achsen jeweils unterschiedlich dimensionierte Eingangsbereiche 31 auf, d.h. die Eingangsbereiche 31 der Markierungen 3, 3a, 3b, 3c, 3d weisen unterschiedliche Längen in der Abtastrichtung (Distanz zwischen Eingangskante 311 und Ausgangskante 312) auf, was sich in unterschiedlichen Zeitwerten T2-T1 und damit verschiedenen Werten der Quadrantenmarker QM auswirkt.

Abschliessend soll festgehalten werden, dass die gezeigten und beschriebenen Signalverläufe bei absorbierenden oder komplementär ausgestalteten Markierungen 3, 3a, 3b, 3c, 3d auch invertiert sein können, wobei die beschriebenen abfallenden respektive ansteigenden Signalflanken dann ansteigend respektive abfallend sind. Weiterhin wird festgehalten, dass die Form der Signalflanken vom Strahlprofil abhängt und dass die obenstehende Beschreibung auf Laserstrahlen mit einem gaussschen Strahlprofil ausgerichtet ist. Laserstrahlen mit nicht gaussförmigem Strahlprofil erzeugen beim Abtasten der Markierungskanten Signalflanken mit anderen Formen. Der Fachmann wird jedoch verstehen, dass in Fällen, wo die Signalflankenform nicht (z.B. analytisch) bekannt ist, in einem Kalibrierungsschritt das Strahlprofil erfasst und die Berechnungen entsprechend angepasst werden können.

**Patentansprüche**

1. Nicht-chirurgisches Verfahren zum Bestimmen der Fokusposition eines Laserstrahls (60, 60a) in einem ophthalmologischen Laserprojektionssystem (1), umfassend:

   Überfahren von mindestens einer auf einer Referenzfläche (20) angebrachten Messmarkierung (3, 3a, 3b, 3c, 3d) mit dem Laserstrahl (60, 60a) entlang einer Abtastbahn (9, 9a, 9b),
   Erfassen eines durch das Überfahren der Messmarkierung (3, 3a, 3b, 3c, 3d) erzeugten Messsignals, **gekennzeichnet, durch** das
   Bestimmen von Zeitwerten (T2, T3, T4, T4') von mindestens einer im Messsignal beim Überfahren von Kanten (311, 312, 321, 322) der Messmarkierung (3, 3a, 3b, 3c, 3d) entstehenden Signalflanke, und
   Ermitteln der Fokusposition basierend auf den Zeitwerten (T2, T3, T4, T4').

2. Verfahren nach Anspruch 1, **dadurch gekennzeichnet, dass** die Messmarkierung (3, 3a, 3b, 3c, 3d) fotoaktiv ausgestaltet wird, und dass beim Überfahren der Messmarkierung (3, 3a, 3b, 3c, 3d) mit dem Laserstrahl (60, 60a) die Messmarkierung (3, 3a, 3b, 3c, 3d) das Messsignal erzeugt.

3. Verfahren nach Anspruch 1, **dadurch gekennzeichnet, dass** die Messmarkierung (3, 3a, 3b, 3c, 3d) reflektierend oder absorbierend ausgestaltet wird, und dass das Messsignal aufgrund eines beim Überfahren der Messmarkierung (3, 3a, 3b, 3c, 3d) reflektierten respektive absorbierten Laserstrahls (60b) erzeugt wird.

4. Verfahren nach einem der Ansprüche 1 bis 3, **dadurch gekennzeichnet, dass** basierend auf den Zeitwerten (T2, T3, T4, T4') eine Strahlbreite (w) bestimmt wird, und dass basierend auf der Strahlbreite (w) die Fokusposition in Projektionsrichtung des Laserstrahls (60, 60a) ermittelt wird.

**5.** Verfahren nach einem der Ansprüche 1 bis 4, **dadurch gekennzeichnet, dass** in der Signalflanke eine Zeitdauer ($\Delta t_1$, $\Delta t_2$) zwischen einem oberen und einem unteren Signalschwellwert bestimmt wird, dass basierend auf der Zeitdauer ($\Delta t_1$, $\Delta t_2$) eine Strahlbreite (w) bestimmt wird, und dass basierend auf der Strahlbreite (w) die Fokusposition in Projektionsrichtung des Laserstrahls (60, 60a) ermittelt wird.

**6.** Verfahren nach einem der Ansprüche 1 bis 5, **dadurch gekennzeichnet, dass** die Messmarkierung (3, 3a, 3b, 3c, 3d) mit einem Spalt (30) respektive einem Balken bekannter Breite ($d_{REF}$) ausgestaltet wird, dass der Spalt (30) respektive der Balken auf einer quer zum Spalt (30) respektive zum Balken verlaufenden Abtastbahn (9, 9a, 9b) überfahren wird, dass aus dem Messsignal eine Zeitdauer ($\Delta t_{ref}$) für das Überfahren der Breite des Spalts (30) respektive des Balkens bestimmt wird, und dass die Fokusposition basierend auf dieser Zeitdauer ($At_{ref}$) und der Breite ($d_{REF}$) des Spalts (30) respektive des Balkens ermittelt wird.

**7.** Verfahren nach einem der Ansprüche 1 bis 6, **dadurch gekennzeichnet, dass** mehrere Messmarkierungen (3a, 3b, 3c, 3d) auf der Referenzfläche (20) auf einem Kreis angeordnet werden, dass die Messmarkierungen (3, 3a, 3b, 3c, 3d) jeweils mit mindestens einer im Wesentlichen senkrecht zum Kreis verlaufenden ersten Kante (321) und einer schräg zu dieser ersten Kante (321) verlaufenden zweiten Kante (322) ausgestaltet werden, dass die kreisförmig angeordneten Messmarkierungen (3a, 3b, 3c, 3d) auf einer kreisförmigen oder spiralförmigen Abtastbahn (9) überfahren werden, dass Zeitwerte (T2, T3, T4, T4') von den im Messsignal beim Überfahren der ersten Kante (321) und der zweiten Kante (322) entstehenden Signalflanken bestimmt werden, und dass aus den Zeitwerten (T2, T3, T4, T4') ein Zentrierkennwert der kreisförmigen oder spiralförmigen Abtastbahn (9) zu den kreisförmig angeordneten Messmarkierungen (3a, 3b, 3c, 3d) ermittelt wird.

**8.** Vorrichtung (2, 10) zum Bestimmen der Fokusposition eines Laserstrahls (60, 60a) in einem ophthalmologischen Laserprojektionssystem (1), umfassend:

einen Körper (2) mit einer Referenzfläche (20) und mindestens einer auf der Referenzfläche (20) angebrachten Messmarkierung (3, 3a, 3b, 3c, 3d),
ein Detektionssystem (4), das eingerichtet ist, ein beim Überfahren der mindestens einen Messmarkierung (3, 3a, 3b, 3c, 3d) mit dem Laserstrahl (60, 60a) entlang einer Abtastbahn (9, 9a, 9b) erzeugtes Messsignal zu erfassen,
**gekennzeichnet durch**
ein Verarbeitungssystem (5), das eingerichtet ist, Zeitwerte (T2, T3, T4, T4') von mindestens einer im Messsignal beim Überfahren von Kanten (311, 312, 321, 322) der Messmarkierung (3, 3a, 3b, 3c, 3d) entstehenden Signalflanke zu bestimmen, und die Fokusposition basierend auf den Zeitwerten (T2, T3, T4, T4') zu ermitteln.

**9.** Vorrichtung (2, 10) nach Anspruch 8, **dadurch gekennzeichnet, dass** die Messmarkierung (3, 3a, 3b, 3c, 3d) fotoaktiv ausgestaltet ist, und dass das Detektionssystem (4) eingerichtet ist, ein beim Überfahren der Messmarkierung (3, 3a, 3b, 3c, 3d) mit dem Laserstrahl (60, 60a) durch die Messmarkierung (3, 3a, 3b, 3c, 3d) erzeugtes Messsignal zu erfassen.

**10.** Vorrichtung (2, 10) nach Anspruch 8, **dadurch gekennzeichnet, dass** die Messmarkierung (3, 3a, 3b, 3c, 3d) reflektierend oder absorbierend ausgestaltet ist, und dass das Detektionssystem (4) eingerichtet ist, das Messsignal aufgrund eines beim Überfahren der Messmarkierung (3, 3a, 3b, 3c, 3d) reflektierten respektive absorbierten Laserstrahls (60b) zu erfassen.

**11.** Vorrichtung (2, 10) nach einem der Ansprüche 8 bis 10, **dadurch gekennzeichnet, dass** das Verarbeitungssystem (5) eingerichtet ist, basierend auf den Zeitwerten (T2, T3, T4, T4') eine Strahlbreite (w) zu bestimmen und basierend auf der Strahlbreite (w) die Fokusposition in Projektionsrichtung des Laserstrahls (60, 60a) zu ermitteln.

**12.** Vorrichtung (2, 10) nach einem der Ansprüche 8 bis 11, **dadurch gekennzeichnet, dass** das Verarbeitungssystem (5) eingerichtet ist, in der Signalflanke eine Zeitdauer ($\Delta t_1$, $\Delta t_2$) zwischen einem oberen und einem unteren Signalschwellwert zu bestimmen, basierend auf der Zeitdauer ($\Delta t_1$, $\Delta t_2$) eine Strahlbreite (w) zu bestimmen, und basierend auf der Strahlbreite (w) die Fokusposition in Projektionsrichtung des Laserstrahls (60, 60a) zu ermitteln.

**13.** Vorrichtung (2, 10) nach einem der Ansprüche 8 bis 12, **dadurch gekennzeichnet, dass** die Messmarkierung (3, 3a, 3b, 3c, 3d) mit einem Spalt (30) respektive einem Balken bekannter Breite ($d_{REF}$) ausgestaltet ist, dass das Detektionssystem (4) eingerichtet ist, ein beim Überfahren des Spalts (30) respektive des Balkens auf einer quer zum Spalt (30) respektive zum Balken verlaufenden Abtastbahn (9, 9a, 9b) erzeugtes Messsignal zu erfassen, und dass das Verarbeitungssystem (5) eingerichtet ist, aus dem Messsignal eine Zeitdauer ($\Delta t_{ref}$) für das Überfahren der Breite ($d_{REF}$) des Spalts respektive des Balkens zu bestimmen, und die Fokusposition basierend auf dieser Zeitdauer ($\Delta t_{ref}$) und der Breite ($d_{REF}$) des Spalts (30) respektive des Balkens zu ermitteln.

14. Vorrichtung (2, 10) nach einem der Ansprüche 8 bis 13, **dadurch gekennzeichnet, dass** mehrere Messmarkierungen (3a, 3b, 3c, 3d) auf der Referenzfläche (20) auf einem Kreis angeordnet sind, dass die Messmarkierungen (3a, 3b, 3c, 3d) jeweils mit mindestens einer im Wesentlichen senkrecht zum Kreis verlaufenden ersten Kante (321) und einer schräg zu dieser ersten Kante (321) verlaufenden zweiten Kante (322) ausgestaltet sind, dass das Detektionssystem (4) eingerichtet ist, ein beim Überfahren der kreisförmig angeordneten Messmarkierungen (3a, 3b, 3c, 3d) auf einer kreisförmigen oder spiralförmigen Abtastbahn (9) erzeugtes Messsignal zu erfassen, und dass das Verarbeitungssystem (5) eingerichtet ist, Zeitwerte (T2, T3, T4, T4') von den im Messsignal beim Überfahren der ersten Kante (321) und der zweiten Kante (322) entstehenden Signalflanken zu bestimmen, und aus den Zeitwerten (T2, T3, T4, T4') ein Zentrierkennwert der kreisförmigen oder spiralförmigen Abtastbahn (9) zu den kreisförmig angeordneten Messmarkierungen (3, 3a, 3b, 3c, 3d) zu bestimmen.

15. Verfahren nach einem der Ansprüche 1 bis 7, **dadurch gekennzeichnet, dass** der Laserstrahl (60, 60a) von einer Laserquelle (6) erzeugt wird, die beim Einleiten eines Messvorgangs zum Bestimmen der Fokusposition des Laserstrahls (60, 60a) in einen Messmodus versetzt wird, wobei die Leistung der Laserquelle (6) auf eine Messleistung gesetzt wird, welche die Messmarkierung (3, 3a, 3b, 3c, 3d) nicht beschädigt oder zerstört.

**Claims**

1. Non-surgical method for determining the focus position of a laser beam (60, 60a) in an ophthalmological laser projection system (1), comprising the steps of:

   passing over at least one measurement marking (3, 3a, 3b, 3c, 3d) applied to a reference area (20) by means of the laser beam (60, 60a) along a scanning path (9, 9a, 9b),
   capturing a measurement signal created by passing over the measurement marking (3, 3a, 3b, 3c, 3d),
   **characterized by**
   determining time values (T2, T3, T4, T4') of at least one signal edge created in the measurement signal when passing over edges (311, 312, 321, 322) of the measurement marking (3, 3a, 3b, 3c, 3d), and
   establishing the focus position on the basis of the time values (T2, T3, T4, T4').

2. Method according to Claim 1, **characterized in that** the measurement marking (3, 3a, 3b, 3c, 3d) has a photoactive design, and **in that** the measurement marking (3, 3a, 3b, 3c, 3d) creates the measurement signal when the laser beam (60, 60a) passes over the measurement marking (3, 3a, 3b, 3c, 3d).

3. Method according to Claim 1, **characterized in that** the measurement marking (3, 3a, 3b, 3c, 3d) has a reflecting or absorbing design, and **in that** the measurement signal is created as a result of a laser beam (60b) which is reflected or absorbed when the measurement marking (3, 3a, 3b, 3c, 3d) is passed over.

4. Method according to one of Claims 1 to 3, **characterized in that** a beam width (w) is determined on the basis of the time values (T2, T3, T4, T4'), and **in that** the focus position in the projection direction of the laser beam (60, 60a) is established on the basis of the beam width (w).

5. Method according to one of Claims 1 to 4, **characterized in that** a time duration ($\Delta t_1$, $\Delta t_2$) between an upper and a lower signal threshold is determined in the signal edge, **in that** a beam width (w) is determined on the basis of the time duration ($\Delta t_1$, $\Delta t_2$), and **in that** the focus position in the projection direction of the laser beam (60, 60a) is established on the basis of the beam width (w).

6. Method according to one of Claims 1 to 5, **characterized in that** the measurement marking (3, 3a, 3b, 3c, 3d) is designed with a gap (30) or bar having a known width ($d_{REF}$), **in that** the gap (30) or bar is passed over on a scanning path (9, 9a, 9b) running across the gap (30) or bar, **in that** a time duration ($\Delta t_{ref}$) for passing over the width of the gap (30) or bar is determined from the measurement signal, and **in that** the focus position is established on the basis of this time duration ($\Delta t_{ref}$) and the width ($d_{REF}$) of the gap (30) or bar.

7. Method according to one of Claims 1 to 6, **characterized in that** a plurality of measurement markings (3a, 3b, 3c, 3d) on the reference area (20) are arranged in a circle, **in that** the measurement markings (3, 3a, 3b, 3c, 3d) are respectively designed with at least one first edge (321) running substantially perpendicular to the circle and a second edge (322) running obliquely to this first edge (321), **in that** the circularly arranged measurement markings (3a, 3b, 3c, 3d) are passed over on a circular or spiral scanning path (9), **in that** time values (T2, T3, T4, T4') are determined from the signal edges created in the measurement signal when passing over the first edge (321) and the second edge (322), and **in that** a centering characteristic of the circular or spiral scanning path (9) with respect to the circularly arranged measurement markings (3a, 3b, 3c, 3d) is established from the time values (T2, T3, T4, T4').

8.   Device (2, 10) for determining the focus position of a laser beam (60, 60a) in an ophthalmological laser projection system (1), comprising:

a body (2) with a reference area (20) and at least one measurement marking (3, 3a, 3b, 3c, 3d) applied to the reference area (20),
a detection system (4), which is designed to capture a measurement signal created when passing over the at least one measurement marking (3, 3a, 3b, 3c, 3d) with the laser beam (60, 60a) along a scanning path (9, 9a, 9b), **characterized by**
a processing system (5), which is designed to determine time values (T2, T3, T4, T4') of at least one signal edge created in the measurement signal when passing over edges (311, 312, 321, 322) of the measurement marking (3, 3a, 3b, 3c, 3d), and to establish the focus position on the basis of the time values (T2, T3, T4, T4').

9.   Device (2, 10) according to Claim 8, **characterized in that** the measurement marking (3, 3a, 3b, 3c, 3d) has a photoactive design, and **in that** the detection system (4) is designed to capture a measurement signal created by the measurement marking (3, 3a, 3b, 3c, 3d) when passing over the measurement marking (3, 3a, 3b, 3c, 3d) with the laser beam (60, 60a).

10.  Device (2, 10) according to Claim 8, **characterized in that** the measurement marking (3, 3a, 3b, 3c, 3d) has a reflecting or absorbing design, and **in that** the detection system (4) is designed to capture the measurement signal on the basis of a laser beam (60b) reflected or absorbed when passing over the measurement marking (3, 3a, 3b, 3c, 3d).

11.  Device (2, 10) according to one of Claims 8 to 10, **characterized in that** the processing system (5) is designed to determine a beam width (w) on the basis of the time values (T2, T3, T4, T4') and to establish the focus position in the projection direction of the laser beam (60, 60a) on the basis of the beam width (w).

12.  Device (2, 10) according to one of Claims 8 to 11, **characterized in that** the processing system (5) is designed to determine a time duration ($\Delta t_1$, $\Delta t_2$) in the signal edge between an upper and a lower signal threshold, to determine a beam width (w) on the basis of the time duration ($\Delta t_1$, $\Delta t_2$), and to establish the focus position in the projection direction of the laser beam (60, 60a) on the basis of the beam width (w).

13.  Device (2, 10) according to one of Claims 8 to 12, **characterized in that** the measurement marking (3, 3a, 3b, 3c, 3d) is designed with a gap (30) or bar of a known width ($d_{REF}$), **in that** the detection system (4) is designed to capture a measurement signal created when passing over the gap (30) or bar on a scanning path (9, 9a, 9b) running across the gap (30) or bar, and **in that** the processing system (5) is designed to determine a time duration ($\Delta t_{ref}$) for passing over the width ($d_{REF}$) of the gap or bar from the measurement signal, and to establish the focus position on the basis of this time duration ($\Delta t_{ref}$) and the width ($d_{REF}$) of the gap (30) or bar.

14.  Device (2, 10) according to one of Claims 8 to 13, **characterized in that** a plurality of measurement markings (3a, 3b, 3c, 3d) on the reference area (20) are arranged in a circle, **in that** the measurement markings (3a, 3b, 3c, 3d) are respectively designed with at least one first edge (321) running substantially perpendicular to the circle and a second edge (322) running obliquely to this first edge (321), **in that** the detection system (4) is designed to capture a measurement signal created when passing over the circularly arranged measurement markings (3a, 3b, 3c, 3d) on a circular or spiral scanning path (9), and **in that** the processing system (5) is designed to determine time values (T2, T3, T4, T4') of the signal edges created in the measurement signal when passing over the first edge (321) and the second edge (322), and to determine from the time values (T2, T3, T4, T4') a centering characteristic of the circular or spiral scanning path (9) with respect to the circularly arranged measurement markings (3a, 3b, 3c, 3d).

15.  Method according to one of Claims 1 to 7, **characterized in that** the laser beam (60, 60a) is generated by a laser source (6), which is changed into a measurement mode during the initiation of a measurement process for determining the focus position of the laser beam (60, 60a), wherein the power of the laser source (6) is set to a measurement power which does not damage or destroy the measurement markings (3, 3a, 3b, 3c, 3d)

**Revendications**

1. Procédé non chirurgical pour la détermination de la position de focalisation d'un faisceau laser (60, 60a) dans un système de projection laser ophtalmologique (1), comprenant :

le passage sur au moins une marque de mesure (3, 3a, 3b, 3c, 3d) - mise en place sur une surface de référence (20) - avec le faisceau laser (60, 60a) le long d'un trajet de balayage (9, 9a, 9b),
la détection d'un signal de mesure produit par le passage sur la marque de mesure (3, 3a, 3b, 3c, 3d),
**caractérisé par**
la détermination de valeurs de temps (T2, T3, T4, T4') d'au moins un flanc de signal se produisant dans le signal de mesure lors du passage sur des bords (311, 312, 321, 322) de la marque de mesure (3, 3a, 3b, 3c, 3d), et
la détermination de la position de focalisation sur la base des valeurs de temps (T2, T3, T4, T4').

2. Procédé selon la revendication 1, **caractérisé en ce que** la marque de mesure (3, 3a, 3b, 3c, 3d) est réalisée de manière photo-active et **en ce que**, lors du passage sur la marque de mesure (3, 3a, 3b, 3c, 3d) avec le faisceau laser (60, 60a), la marque de mesure (3, 3a, 3b, 3c, 3d) produit le signal de mesure.

3. Procédé selon la revendication 1, **caractérisé en ce que** la marque de mesure (3, 3a, 3b, 3c, 3d) est réalisée de manière réfléchissante ou absorbante, et **en ce que** le signal de mesure est produit en raison d'un faisceau laser (60b) réfléchi ou absorbé lors du passage sur la marque de mesure (3, 3a, 3b, 3c, 3d).

4. Procédé selon l'une des revendications 1 à 3, **caractérisé en ce que** l'on détermine une largeur de faisceau (w) sur la base des valeurs de temps (T2, T3, T4, T4'), et **en ce que** l'on détermine la position de focalisation en direction de projection du faisceau laser (60, 60a) sur la base de la largeur du faisceau (w).

5. Procédé selon l'une des revendications 1 à 4, **caractérisé en ce que** l'on détermine, dans le flanc de signal, une durée ($\Delta t_1$, $\Delta t_2$) comprise entre une valeur de seuil de signal supérieure et inférieure, **en ce que**, sur la base de la durée ($\Delta t_1$, $\Delta t_2$), on détermine une largeur de faisceau (w) et **en ce que**, sur la base de la largeur de faisceau (w), on détermine la position de focalisation en direction de projection du faisceau laser (60, 60a).

6. Procédé selon l'une des revendications 1 à 5, **caractérisé en ce que** la marque de mesure (3, 3a, 3b, 3c, 3d) est réalisée avec un écartement (30) ou une barre de largeur connue ($d_{REF}$), **en ce que** l'on passe sur l'écartement (30) ou la barre sur un trajet de balayage (9, 9a, 9b) s'étendant transversalement à l'écartement (30) ou à la barre, **en ce que** l'on détermine, à partir du signal de mesure, une durée ($\Delta t_{ref}$) pour le passage sur la largeur de l'écartement (30) ou de la barre, et **en ce que** l'on détermine la position de focalisation sur la base de cette durée ($\Delta t_{ref}$) et de la largeur ($d_{REF}$) de l'écartement (30) ou de la barre.

7. Procédé selon l'une des revendications 1 à 6, **caractérisé en ce que** plusieurs marques de mesure (3a, 3b, 3c, 3d) sont disposées sur un cercle sur la surface de référence (20), **en ce que** les marques de mesure (3, 3a, 3b, 3c, 3d) sont respectivement réalisées avec au moins un premier bord (321) s'étendant sensiblement perpendiculairement au cercle et avec un deuxième bord (322) s'étendant en biais par rapport à ce premier bord (321), **en ce que** l'on passe sur les marques de mesure (3a, 3b, 3c, 3d) disposées en forme de cercle sur un trajet de balayage (9) en forme de cercle ou en forme de spirale, **en ce que** l'on détermine des valeurs de temps (T2, T3, T4, T4') des flancs de signaux produits dans le signal de mesure lors du passage sur le premier bord (321) et le deuxième bord (322), et **en ce que** l'on détermine, à partir des valeurs de temps (T2, T3, T4, T4'), une valeur caractéristique de centrage du trajet de balayage (9) en forme de cercle ou en forme de spirale pour les marques de mesure (3a, 3b, 3c, 3d) disposées en forme de cercle.

8. Dispositif (2, 10) pour la détermination de la position de focalisation d'un faisceau laser (60, 60a) dans un système de projection laser ophtalmologique (1), comprenant :

un corps (2) avec une surface de référence (20) et au moins une marque de mesure (3, 3a, 3b, 3c, 3d) mise en place sur la surface de référence (20),
un système de détection (4) qui est étudié pour détecter un signal de mesure produit lors du passage sur l'au moins une marque de mesure (3, 3a, 3b, 3c, 3d) avec le faisceau laser (60, 60a) le long d'un trajet de balayage (9, 9a, 9b),

**caractérisé par**

un système de traitement (5) qui est étudié pour déterminer des valeurs de temps (T2, T3, T4, T4') d'au moins un flanc de signal se produisant dans le signal de mesure lors du passage sur des bords (311, 312, 321, 322) de la marque de mesure (3, 3a, 3b, 3c, 3d), et pour déterminer la position de focalisation sur la base des valeurs de temps (T2, T3, T4, T4').

9. Dispositif (2, 10) selon la revendication 8, **caractérisé en ce que** la marque de mesure (3, 3a, 3b, 3c, 3d) est réalisée de manière photo-active et **en ce que** le système de détection (4) est étudié pour détecter un signal de mesure produit par la marque de mesure (3, 3a, 3b, 3c, 3d) lors du passage sur la marque de mesure (3, 3a, 3b, 3c, 3d) avec le faisceau laser (60, 60a).

10. Dispositif (2, 10) selon la revendication 8, **caractérisé en ce que** la marque de mesure (3, 3a, 3b, 3c, 3d) est réalisée de manière réfléchissante ou absorbante et **en ce que** le système de détection (4) est étudié pour détecter le signal de mesure en raison d'un faisceau laser (60b) réfléchi ou absorbé lors du passage sur la marque de mesure (3, 3a, 3b, 3c, 3d).

11. Dispositif (2, 10) selon l'une des revendications 8 à 10, **caractérisé en ce que** le système de traitement (5) est étudié pour déterminer une largeur de faisceau (w) sur la base des valeurs de temps (T2, T3, T4, T4') et pour déterminer la position de focalisation en direction de projection du faisceau laser (60, 60a) sur la base de la largeur du faisceau (w).

12. Dispositif (2, 10) selon l'une des revendications 8 à 11, **caractérisé en ce que** le système de traitement (5) est étudié pour déterminer, dans le flanc de signal, une durée ($\Delta t_1$, $\Delta t_2$) comprise entre une valeur de seuil de signal supérieure et inférieure, pour déterminer une largeur de faisceau (w) sur la base de la durée ($\Delta t_1$, $\Delta t_2$), et pour déterminer la position de focalisation en direction de projection du faisceau laser (60, 60a) sur la base de la largeur de faisceau (w).

13. Dispositif (2, 10) selon l'une des revendications 8 à 12, **caractérisé en ce que** la marque de mesure (3, 3a, 3b, 3c, 3d) est réalisée avec un écartement (30) ou une barre de largeur connue ($d_{REF}$), **en ce que** le système de détection (4) est étudié pour détecter un signal de mesure produit lors du passage sur l'écartement (30) ou la barre sur un trajet de balayage (9, 9a, 9b) s'étendant transversalement à l'écartement (30) ou à la barre, et **en ce que** le système de traitement (5) est étudié pour déterminer, à partir du signal de mesure, une durée ($\Delta t_{ref}$) pour le passage sur la largeur ($d_{REF}$) de l'écartement ou de la barre, et pour déterminer la position de focalisation sur la base de cette durée ($\Delta t_{ref}$) et de la largeur ($d_{REF}$) de l'écartement (30) ou de la barre.

14. Dispositif (2, 10) selon l'une des revendications 8 à 13, **caractérisé en ce que** plusieurs marques de mesure (3a, 3b, 3c, 3d) sont disposées sur un cercle sur la surface de référence (20), **en ce que** les marques de mesure (3a, 3b, 3c, 3d) sont respectivement réalisées avec au moins un premier bord (321) s'étendant sensiblement perpendiculairement au cercle et avec un deuxième bord (322) s'étendant en biais par rapport à ce premier bord (321), **en ce que** le système de détection (4) est étudié pour détecter un signal de mesure produit lors du passage sur les marques de mesure (3a, 3b, 3c, 3d) disposées en forme de cercle sur un trajet de balayage (9) en forme de cercle ou en forme de spirale, et **en ce que** le système de traitement (5) est étudié pour déterminer des valeurs de temps (T2, T3, T4, T4') des flancs de signaux produits dans le signal de mesure lors du passage sur le premier bord (321) et le deuxième bord (322) et pour déterminer, à partir des valeurs de temps (T2, T3, T4, T4'), une valeur caractéristique de centrage du trajet de balayage (9) en forme de cercle ou en forme de spirale pour les marques de mesure (3, 3a, 3b, 3c, 3d) disposées en forme de cercle.

15. Procédé selon l'une des revendications 1 à 7, **caractérisé en ce que** le faisceau laser (60, 60a) est produit par une source laser (6) que l'on fait passer à un mode de mesure lors du lancement d'un processus de mesure pour déterminer la position de focalisation du faisceau laser (60, 60a), dans lequel la performance de la source laser (6) est fixée à une performance de mesure qui n'endommage pas ou ne détruit pas la marque de mesure (3, 3a, 3b, 3c, 3d).

**Fig. 2**

**Fig. 1**

**Fig. 3**

**Fig. 4**

**Fig. 5**

**Fig. 6**

**Fig. 7**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10349296

- EP 2069099 A